# EUROPEAN PATENT APPLICATION

(11) **EP 1 547 515 A1**
(43) Date of publication of application: **29.06.2005**
(21) Application number: 03258132.4
(22) Date of filing: 22.12.2003
(51) Int. Cl.: A61B 5/00

(54) **Optical fibre catheter pulse oximeter**

(71) Applicant: Barts and The London National Health Service Trust, Whitechapel, London E1 1BB (GB); QUEEN MARY AND WESTFIELD COLLEGE, London E1 4NW (GB)
(72) Inventor: The designation of the inventor has not yet been filed
(74) Representative: Raynor, John

(57) **Abstract**

Apparatus for measuring the oxygen saturation level of blood at an internal measurement site in a human or animal patient by reflectance pulse oximetry, comprising first and second light sources (5,10), a first optical fibre (30) for transferring light from at least one said light source to the internal measurement site (35), at least one receiver (45), for receiving light from the first and second light sources, at least a second optical fibre (40) for transferring light reflected from the region of the measurement site to the receiver, and means for determining the oxygen saturation level of the blood at the internal measurement site, based on the light produced by the light sources and light received by the receiver. The optical centres of the optical fibres are separated from one another by at least 1mm at their distal ends. The apparatus can be used in combination with a cranial axis bolt for measuring the oxygen saturation level in the brain tissue of a human or animal patient.

## Description

The invention relates to an optical fibre catheter pulse oximeter, and in particular to the use of such oximeters to measure brain tissue oxygen saturation.

Patients suffering from head injuries and those recovering from certain types of neurosurgery are often susceptible to problems with cerebral perfusion (i.e., blood supply to the brain). A shortage of oxygenated blood to the brain rapidly results in ischaemia and in severe cases, death. The brain is perfused to a greater extent than any other organ receiving about 20% of the resting cardiac output. In extreme pathophysiological states the blood supply to other organs is cut off in order to preserve the cerebral circulation. Clearly an effective means of monitoring cerebral oxygenation is invaluable in such situations.

The brain is completely enclosed within the cranial cavity, which presents difficulties with any physical monitoring system.

Several methods have been developed to measure parameters related to brain tissue oxygenation.

Near Infrared Spectroscopy (NIRS) is available commercially but has not yet gained widespread acceptance as a reliable indicator of brain oxygenation. The method provides an estimation of the overall mixed venous oxygen saturation of a region of brain tissue. The method relies on the fact that infrared light can penetrate the skull to some extent although there is considerable attenuation. Optical fibre bundles are used to transmit light from a multiple wavelength source to a sensor usually placed on the forehead. The re-emitted light is sampled by a second optical fibre bundle and returned to a photodetector. An incandescent light source is used with filters to select the wavelengths required.

This method can give estimations of saturation, haemoglobin, oxyhaemoglobin and cytochrome aa₃ concentration. Preterm neonates are ideal candidates for NIRS. Term infants, older children and adults have thicker skulls, which limit the transmission of photons. Presently available NIRS instruments have a number of other limitations, including the measurements being prone to movement artefact and the behaviour of light at tissue boundaries being poorly understood. The proportion of light reflected from the superficial layers (scalp, skull etc.) rather than the light reemitted from the brain is uncertain, making calibration of the system difficult (McCormick, P. W., M. Stewart, et al. (1991) Critical Care Medicine 19(1): 89-97).

Venous Jugular Bulb Oximetry (SjvO₂) is a further technique which is in clinical use, and for which systems are commercially available. It relies on measurement of the oxygen saturation of the venous blood draining from the brain, by the placement of a fibre optic catheter in the bulbous dilatation of the jugular vein at the base of the skull. Continuous readings of venous oxygen saturation are possible, which gives an indication of cerebral oxygen uptake. The major drawback of SjvO₂ is its inability to detect regional ischemia (Gopinath, S. P., A. B. Valadka, et al. (1999) Critical Care Medicine 27(11): 2337-2345).

Brain Tissue pO₂ Sensors (pbtO₂) are a relatively new development that has recently become commercially available (LiCox system, Integra Neurosciences Inc.). A miniature electrochemical sensor (Clark electrode) is inserted into the brain via a hollow bolt screwed into the skull. The partial pressure of oxygen dissolved in the intracellular fluid is measured. PbtO2 is a useful indicator of local tissue oxygenation as only the tissue in direct contact with the sensor is monitored. Researchers have found that small haemorrhages sometimes form around the catheter tip causing unreliable results to be recorded. Furthermore it often takes several hours for the readings to stabilize (Gopinath, Valadka et al. 1999).

Pulse oximetry for the measurement of oxygen saturation levels of blood is one of the most common measurements carried out in current medical practice. The technique involves the use of two light sources of different wavelengths (typically 660nm and 850nm), which are used to illuminate a region of tissue. The two wavelengths are differentially absorbed by haemoglobin, by amounts which differ depending on whether the haemoglobin is saturated or desaturated with oxygen. Light passing through or reflected from the tissue is detected, and the absorption at the two wavelengths is compared in order to compute the proportion of haemoglobin which is oxygenated.

The term "pulse oximetry" is derived from the fact that signal variation is detected in phase with the pulsing of the blood flow of the patient, in order to distinguish the signal due to pulsatile flow from other more static signals.

In transmission pulse oximetry, which is by far the most common method of operation, the measurement points generally selected for pulse oximetry measurements are peripheral points of the body which are easily accessible, such as the fingers and ear-lobes. The results obtained by such measurements are acceptable for most purposes. These measurement sites are however not suitable when certain medical conditions are present, for example peripheral circulatory impairment, resulting in poor circulation in the fingers.

Reflectance pulse oximeter probes for placement on the skin (for example on the forehead) are also commercially available. These devices offer an alternative method of monitoring oxygenation in patients with compromised peripheral perfusion or low peripheral skin temperature. However, they are not able to measure blood perfusion in inaccessible regions of the body, such as the brain.

Various attempts have been made to extend the range of pulse oximetry to internal organs. In particular Kyriacou et al. (2003 "Evaluation of oesophageal pulse oximetry in patients undergoing cardiothoracic surgery" Anaesthesia 58: 422-427) have developed a device for measuring the oxygen saturation of internal tissue in the oesophagus, using reflectance pulse oximetry. The probe proposed by Kyriacou consists of LEDs and photodiodes, is approximately 3.5mm in diameter and is housed in an oesophageal probe of 6mm approximate outside diameter. The probe is intended for insertion after anaesthetic induction prior to surgery, where it remains until the early recovery phase.

WO-A-0013575 discloses a device taking pulse oximeter readings within the nasal and oral cavities, the surface of the posterior pharynx and the interior of the ear canal. The probe is of a similar general type to the Kyriacou probe, with the light source being positioned in the immediate vicinity of the tissue under investigation.

We have now found that useful oxygenation readings may be obtained in a significantly increased number of clinical situations, in particular of internal body tissue, by providing a reflectance pulse oximeter, in which a light source is positioned remote from the tissue under investigation, and light is transferred from the light source to the measurement site by means of an optical fibre.

We have also found that apparatus of this general kind is particularly suited to the measurement of oxygen saturation levels in the brain.

In a first aspect of the invention, there is provided apparatus for measuring the oxygen saturation level of blood at an internal measurement site in a human or animal patient by reflectance pulse oximetry, comprising a first light source having a first spectral distribution, a second light source having a second spectral distribution, a first optical fibre having a proximal end adjacent at least one said light source, and a distal end adapted in use to be positioned adjacent said internal measurement site, for transferring light from at least one said light source to the internal measurement site, at least one receiver, for receiving light from the first and second light sources, at least a second optical fibre having a proximal end adjacent the said receiver, and a distal end adapted in use to be positioned adjacent said internal measurement site, for transferring light reflected from the region of the measurement site to the receiver, means for determining the oxygen saturation level of the blood at the internal measurement site, based on the light produced by the light sources and light received by the receiver, wherein the optical centres of the first and second optical fibres are separated from one another by at least 1 mm at their distal ends.

Preferably the light sources are monochromatic.

The use of an optical fibre to convey light to and from the internal measurement site enables the intrusive part of the apparatus (i.e. that which lies within the patient in use) to be both very flexible, and small in diameter. The apparatus therefore facilitates the measurement of oxygenation in regions which would otherwise be inaccessible, in particular in the oesophagus, trachea, and colon, and in particular, in the region of the surface of the brain.

Because of the use of the optical fibres, the electrical components and the light sources can be maintained externally of the patient, out of contact with patient tissue, thereby reducing the likelihood of bums to the patient, as well as rendering the apparatus less intrusive.

The apparatus may be usefully employed at any internal measurement site at which it is desired to measure blood oxygenation. In many cases, only a very small incision is required for the device to be positioned.

Separate optical fibres may be utilised in order to transfer light from each of the two light sources to the internal measurement site. In a preferred embodiment however light from the two light sources may be fed down a single optical fibre. In a particularly preferred embodiment, light from the two light sources may be pulsed alternately (i.e., sequentially) down a single optical fibre. Similarly, a single optical fibre may be employed for collecting light reflective from the internal measurement site, and transmitting it to the receiver. The use of single optical fibres in this way enables the overall dimensions, in particular the diameter of the device to be minimised.

The optical fibres are preferably separated from each other by means of a spacer optical fibre positioned between the transfer optical fibres.

The first light source preferably produces light in the red part of the visible spectrum, and the second light source preferably produces light in the near-infrared part of the spectrum. Preferred peak emission wavelengths are from 630 nm to 760 nm for the red light and from 820 nm to 930 nm for the infrared light. Particularly preferred peak emission wavelengths of 660 nm and 850 nm were found to be effective for the red and infrared light sources respectively although other wavelengths near these values could also be used. It is preferable that the light sources are either monochromatic or at least emit light over a narrow wavelength band. In a preferred embodiment, the light sources are Light Emitting Diodes (LEDs), which typically have a spectral line half-width of approximately 5 % of the peak emission wavelength value.

The apparatus according to the invention is particularly suited to the measurement of the oxygenation saturation level of blood in brain tissue. A cranial access bolt, which may be of generally conventional form, may be provided to support the distal ends of the optical fibres at the appropriate position, spaced from the brain surface. The cranial access bolt is preferably such that the distal ends of the optical fibres are supported at a distance of from 0 to 4.0 mm from the tissue surface. The cranial access bolt may also be used to facilitate the positioning other measurement devices.

In a further aspect of the invention, there is provided a method of measuring the oxygen saturation level in the brain tissue of a human or animal patient, comprising the steps of inserting the distal ends of the optical fibres of apparatus as described above through a cranial access bolt positioned in the skull of the patient, positioning the distal ends of the optical fibres at a distance of from 0 to 4.0 mm from the brain surface, illuminating the brain surface of the patient using the said light sources, and determining the oxygen saturation level of blood at the brain surface from reflected light received at the receiver via the said second optical fibre.

The device according to the invention permits blood oxygenation, which has proved to be an effective clinical indicator, to be measured directly on brain tissue. The method offers the possibility of improved reliability, as compared with NIRS, because it generally suffers less from scattering and absorption of the light by superficial layers.

Furthermore, optical measurements have the advantage that monitoring can begin as soon as the device is in place (i.e. there is no settling-in time as in with the pO2 monitor). The optical fibre sensor is also less invasive than a pO2 electrode, which has to be inserted several centimetres into the brain tissue, which can cause tissue damage which can in turn lead to unreliable measurement.

A preferred embodiment of the invention will be further described with reference to the following figures in which:
Figure 1 shows a schematic diagram of one embodiment of apparatus according to the invention.
Figure 2 shows a schematic layout of the arrangement at the proximal end of the apparatus of Figure 1.
Figure 3 shows a cross sectional view of the distal tips of the optical fibres of the apparatus of Figure 1.
Figure 4 shows Cranial access bolt supporting the optical fibres of apparatus according to an embodiment of the invention.
Figure 5 shows an LED timing cycle suitable for use in the apparatus of Figure 1.
Figure 6 shows a preferred arrangement of the positioning of the tips of the optical fibres of the apparatus of Figure 1, relative to an internal measurement site.
Figure 7 shows the apparatus of Figure 4, in position in the skull of a patient..

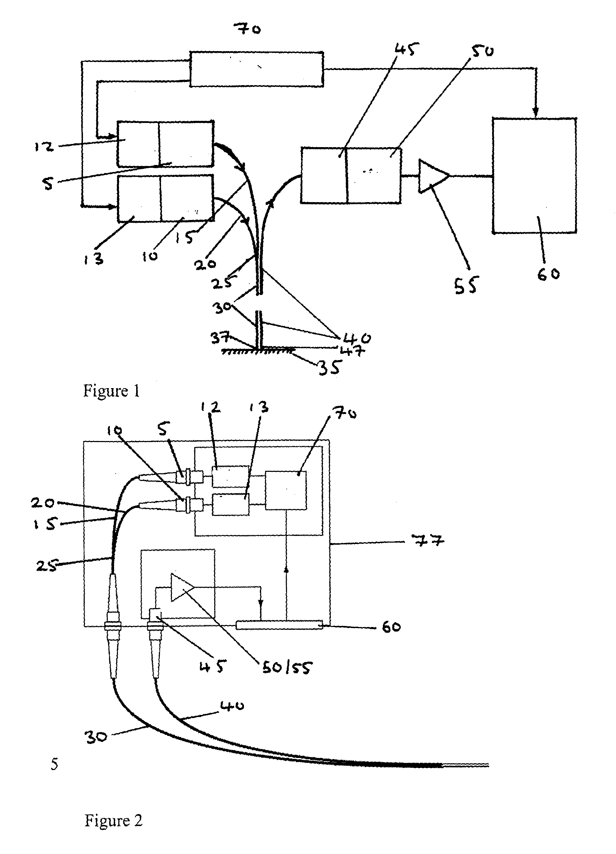

The apparatus of Figure 1 comprises a first light source (5), in the form of an LED which emits pulses of red light at a wavelength of 660 nm, and a second light source (10), which is an LED with a wavelength of 850 nm (in the infrared). The light sources (5,10) are connected to current sources (12, 13) for driving the light source. Optical fibres (15, 20) are connected between light sources (5, 10) and the upper limbs of a "Y"-coupler (not shown, but indicated generally at location 25). The "Y"-coupler connects optical fibres (15, 20) with a single optical fibre (30) for transferring light to the distal end (37) of the device, positioned adjacent an internal measurement site (35) in a human or animal patient.

A further optical fibre (40) is provided for transferring light reflected from the measurement site (35) to a photodiode receiver (45). The optical fibre (40) has an end (47) for positioning near to the measurement site to receive the emitted light. The receiver (45) is connected to a transimpedence amplifier (50), for converting the photocurrent from the receiver to a voltage. The transimpedence amplifier is connected via a further amplifier (55) for amplifying the resultant voltage, to an interface (60), for converting the signal from digital to analogue, and demultiplexing the signal, to allow a value for oxygen saturation to be obtained.

A logic circuit (70) having a system clock and a counter timer is connected to the light sources (5, 10), and the interface (60). The logic circuit (70) can produce a timing cycle for producing a multiplexed signal of light pulses from the light sources (5, 10), and for enabling the interface (60) to demultiplex the signals received by the receiver (45).

Light sources (5, 10), logic circuit (70), current sources (12, 13), photodetector (45), transimpedence amplifier (50), amplifier (55) and interface (60) are housed in a casing (77) which is shown schematically in Figure 2. The circuit is powered by two 12V lead-acid cells (not shown).

The optical fibres (15, 20, 40) are coupled to the light sources (5, 10) by communications industry-standard SMA connectors to minimise energy losses at each connection.

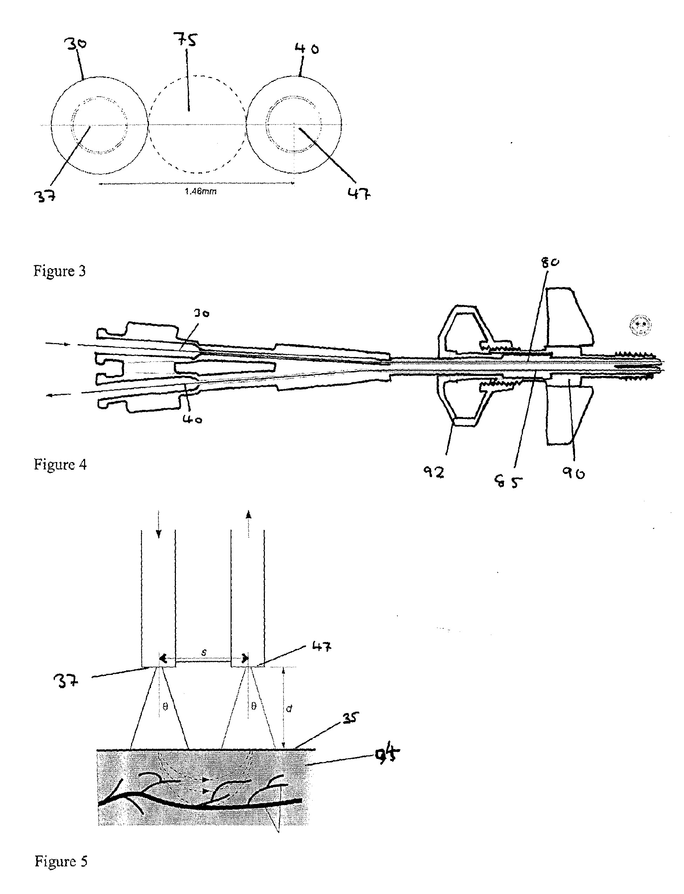

The optical fibres (30, 40) are connected together so that the optical centres of the distal ends (37, 47) of the optical fibres (30, 40) are separated from each other by 1.46 mm, as shown in Figure 3. The ends (37, 47) are separated by a spacer fibre (75) of approximately the same diameter as the optical fibres (30, 40). The ends (37, 47) are cleaved and polished to form a flat face to ensure uniform transmission of light out of and into the fibres.

Each optical fibre (15, 20, 30, 40) has an outer diameter of 730µm and a core diameter of 400µm. The acceptance angle (θ), i.e. the maximum angle for receiving light, of each fibre is 23°. The fibre is made of hard-clad silica, which is sterilisable and fully biocompatible.

Each of the optical fibres (30, 40) can be threaded through a channel (80, 85) in a cranial access bolt (90) such as the LiCox® IM3 cranial access system manufactured by Integra Neurocare LLC, San Diego, CA. USA, as shown in Figure 4. The bolt has three channels, leaving one channel free for a further sensor if required.

Once the fibres (30, 40) are in the correct position in the channels (80, 85), a compression cap (92) can be tightened to create a sterile seal to prevent contamination of internal tissue.

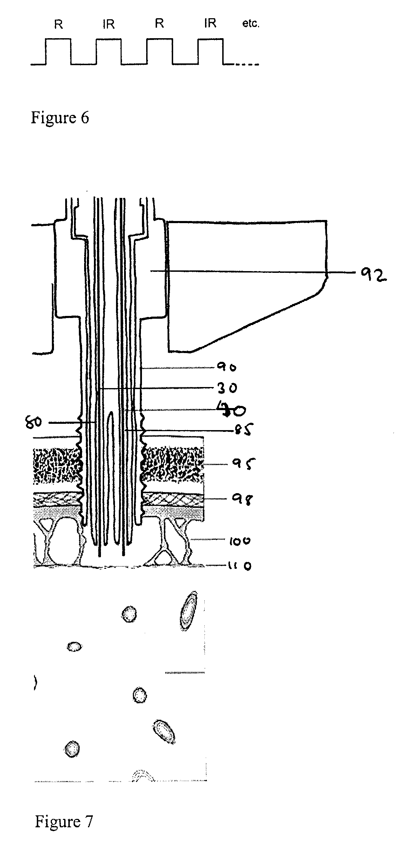

In use, the ends (37, 47) of the fibres (30, 40) are positioned near to the internal measurement site (35) of the patient. The ends (37, 47) can be positioned close to, touching or penetrating the surface of a patient's tissue in order to be positioned correctly for the internal measurement site (35). The optimal distance d, as shown in Figure 5, between the ends (37, 47), and the internal measurement site (35) is 0 < d < s / 2 tan θ, where s is the separation between the optical centres of the fibres (30, 40), and θ is the acceptance angle (23° in this case). However, d should preferably be no greater than 2 mm as at distances greater than this, the detected light is partially reflected from the tissue surface (35) and not wholly scattered within the tissue (94) (a phenomenon known as optical shunt).

The counter timer of the logic circuit (70) produces a dedicated pulse train to allow the light sources (5,10) to pulse sequentially by generating timing signals, which trigger the logic circuit (70), generating the timing cycle shown in Figure 6. The timing cycle produces a multiplexed signal of sequential of red and infra-red pulses.

The pulses pass down the optical fibres (15, 20, 30) to the internal measurement site (35). Each light pulse is wholly scattered within the tissue of the measurement site (35). A portion of the light from each pulse is then emitted from the measurement site (35), where it passes into optical fibre (40). The light then passes along the optical fibre (40) to the photodiode receiver (45). The photodiode receiver (45) generates a current which is directly proportional to the light intensity measured by the receiver (45). The transimpedence amplifier (50) linearly converts the current into a voltage, which is amplified by the amplifier (55). The amplified voltage passes to the interface (60), where it is sampled by a 16 bit digital-to-analogue converter. The logic circuit (70) gates the data acquisition, synchronising the multiplexed pulses and the acquired data. The resultant signals from the digital-to-analogue converter can then be separated by a demultiplexer into separate signals relating to each red and infra red pulse. The signals are individually filtered to remove high-frequency noise.

The oxygen saturation can be calculated from the ratio (R) of the signals relating to the red and infra red pulses, using the formula:

R = (I_{L,R} / I_{H,R}) / (I_{L,IR} / I_{H,IR})

wherein I_{L,R} and I_{H,R} are the lowest and highest values respectively of the light intensity detected during the 'ON' phases of the red light source, during one cardiac cycle. I_{L,IR} and I_{H,IR} are the corresponding values for the light intensity detected during the 'ON' phases of the infrared sources.

The oxygen saturation (SpO₂) can be estimated from an empirically derived calibration curve, for example using first order equations.

Figure 7 illustrates the use of the pulse oximeter of Figure 1 in combination with a LiCox® cranial bolt, to measure blood oxygenation levels at the surface of the brain. The bolt (90) is screwed through the skull (95) and the dura mater (98). Optical fibres (30, 40) are passed through two of the three channels (80, 85), typically the temperature and oxygen electrode channels, until they are in position in the arachnoid mater (100), which allows measurement of the oxygenation level of the blood in the pia mater (110). The compression cap (92) is then tightened to create the sterile seal, and the blood oxygenation level can be measured.

## Claims

1. Apparatus for measuring the oxygen saturation level of blood at an internal measurement site in a human or animal patient by reflectance pulse oximetry, comprising:
a first light source having a first spectral distribution;
a second light source having a second spectral distribution;
a first optical fibre having a proximal end adjacent at least one said light source, and a distal end adapted in use to be positioned adjacent said internal measurement site, for transferring light from at least one said light source to the internal measurement site; at least one receiver, for receiving light from the first and second light sources;
at least a second optical fibre having a proximal end adjacent the said receiver, and a distal end adapted in use to be positioned adjacent said internal measurement site, for transferring light reflected from the region of the measurement site to the receiver; means for determining the oxygen saturation level of the blood at the internal measurement site, based on the light produced by the light sources and light received by the receiver,
wherein the optical centres of the first and second optical fibres are separated from one another by at least 1 mm at their distal ends.

2. Apparatus as claimed in claim 1, wherein the first light source is monochromatic.

3. Apparatus as claimed in claim 1 or claim 2, wherein the second light source is monochromatic.

4. Apparatus as claimed in any one of claims 1 to 3, including means for pulsing light from the first and second light sources sequentially along the first optical fibre.

5. Apparatus as claimed in any one of the preceding claims, wherein the first light source is such as to produce light having a peak emission wavelength of from 630 nm to 760 nm and the second light source is such as to produce light having a peak emission wavelength of from 820 nm to 930 nm.

6. Apparatus as claimed in any one of the preceding claims, wherein the said measurement site is the surface of the brain, and wherein the apparatus additionally comprises a cranial access bolt for insertion into the skull of the patient, and means for supporting the said optical fibres in the access bolt, such that light from the said light sources is directed towards the surface of the brain, thereby enabling measurement of the oxygen saturation level of blood at the brain surface.

7. Apparatus as claimed in claim 6, wherein the cranial access bolt is adapted to support the said optical fibres, such that the distal ends of the optical are positioned from 0 to 4.0 mm from the surface of the brain.

8. A method of measuring the oxygen saturation level in the brain tissue of a human or animal patient, comprising the steps of inserting the distal ends of the optical fibres of apparatus as claimed in any one of claims 1 to 5 through a cranial access bolt positioned in the skull of the patient;
positioning the distal ends of the optical fibres at a distance of from 0 to 4.0 mm from the brain surface;
illuminating the brain surface of the patient using the said light sources;
and determining the oxygen saturation level of blood at the brain surface from reflected light received at the receiver via the said second optical fibre.
